# EUROPEAN PATENT APPLICATION

(11) **EP 4 549 002 A2**
(43) Date of publication of application: **07.05.2025**
(21) Application number: 24206630.6
(22) Date of filing: 15.10.2024
(51) Int. Cl.: B01D 53/86

(54) **METHODS AND SYSTEMS FOR PHOTOCATLYTIC OXIDATION OF WASTE ANESTHETIC GAS**

(30) Priority: 01.11.2023 US 202318499989
(71) Applicant: GE Precision Healthcare LLC, Waukesha, WI 53188 (US)
(72) Inventor: KUZELKA, Russell J., Madison, 53718-6704 (US)
(74) Representative: Kilburn & Strode LLP

(57) **Abstract**

Methods and systems are provided for treating waste anesthetic gas. One method includes flowing (710) waste anesthetic gas into an inlet of a photocatalytic oxidizing reactor while operating one or more ultraviolet lamps positioned inside the photocatalytic oxidizing reactor in order to trigger oxide catalyzed photodecomposition of the waste anesthetic gas and also forming acidic byproducts in the process. An output of the photocatalytic oxidizing reactor includes the acidic byproducts and is flowed (722) to the neutralization reactor.

## Description

### TECHNICAL FIELD

Embodiments of the subject matter disclosed herein relate to systems and methods for photocatalytic oxidation of waste anesthetic gas.

### BACKGROUND

During a surgical procedure, a closed loop anesthesia circuit may be used to deliver a gas mixture, including oxygen and anesthetic agents, to a patient and to collect exhaled gases which include carbon dioxide in addition to excess anesthetic agents. A scavenging system may be configured to collect the exhaled carbon dioxide and excess anesthetic agents in a bag or canister to prevent exposure of the medical personnel in the operating theater to the anesthetic agents. The bag or canister may then be vented outside the building. However, anesthetic agents may include potent greenhouse gases, such as desflurane, N₂O, isoflurane, and sevoflurane, and venting directly to the atmosphere may not be desirable for environmental reasons.

### BRIEF DESCRIPTION

In one embodiment, a method for treating waste anesthetic gas comprises flowing waste anesthetic gas into an inlet of a photocatalytic oxidizing reactor, operating one or more ultraviolet lamps of the photocatalytic oxidizing reactor to trigger oxide catalyzed photodecomposition of waste anesthetic gas and forming acidic byproducts, and flowing an output of the photocatalytic oxidizing reactor, including the acidic byproducts into a neutralization reactor.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present invention will be better understood from reading the following description of non-limiting embodiments, with reference to the attached drawings, herein below:
FIG. 1 is an illustration of a closed loop anesthesia circuit coupled to a photocatalytic waste anesthetic gas treatment system;
FIG. 2 is an illustration of photocatalytic activity of a wide bad semiconductor oxide;
FIG. 3 is an illustration of a first embodiment of a photocatalytic waste anesthetic gas treatment system;
FIG. 4 is an illustration of a second embodiment of a photocatalytic waste anesthetic gas treatment system;
FIG. 5 is an illustration of an alternate embodiment of a photocatalytic waste anesthetic gas reactor;
FIG. 6 is a cross sectional view of the photocatalytic waste anesthetic gas reactor of FIG. 5;
FIG. 7 is a flowchart of a method of operating a photocatalytic waste anesthetic gas reactor of a photocatalytic waste anesthetic gas treatment system;
FIG. 8 is a flow chart of a method of operating a neutralization reactor of a photocatalytic waste anesthetic gas treatment system;
FIG. 9 is a continuation of the method of FIG. 8; and
FIG. 10 is a flow chart of a method of operating a liquid replacement system fluidly coupled to a neutralization reactor.

### DETAILED DESCRIPTION

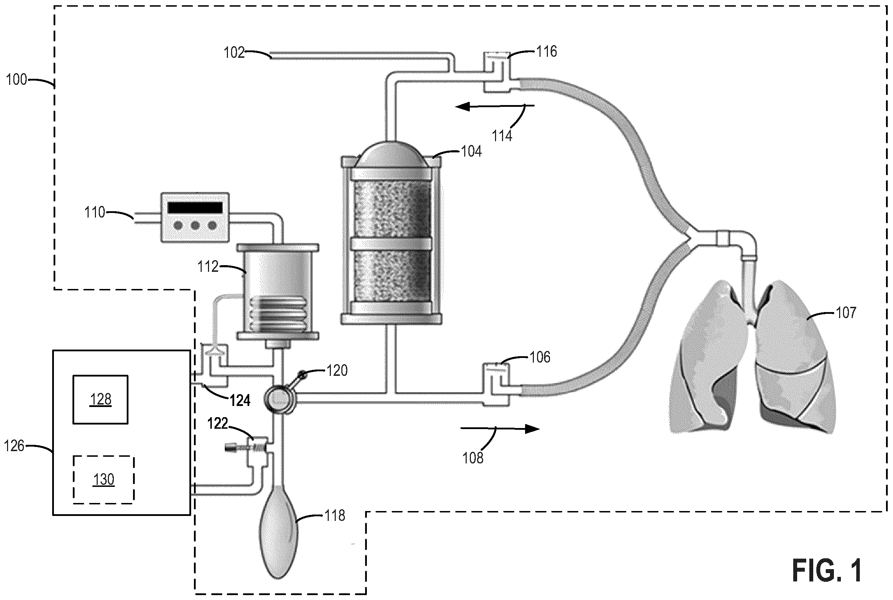

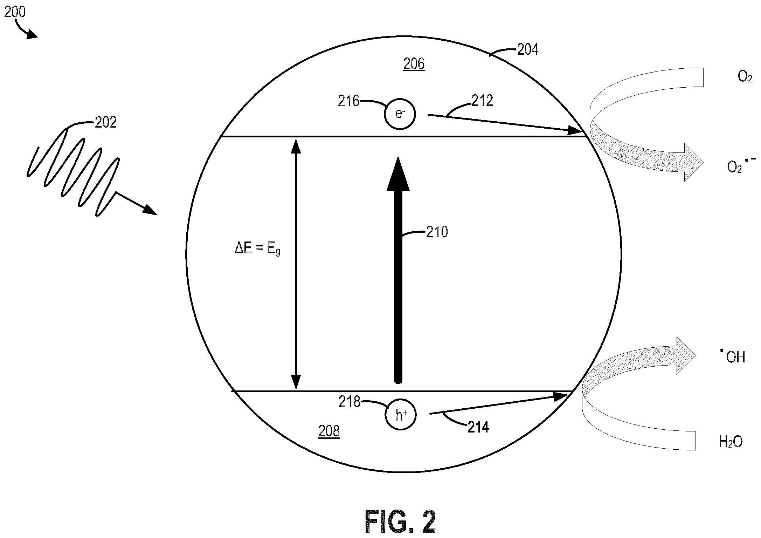

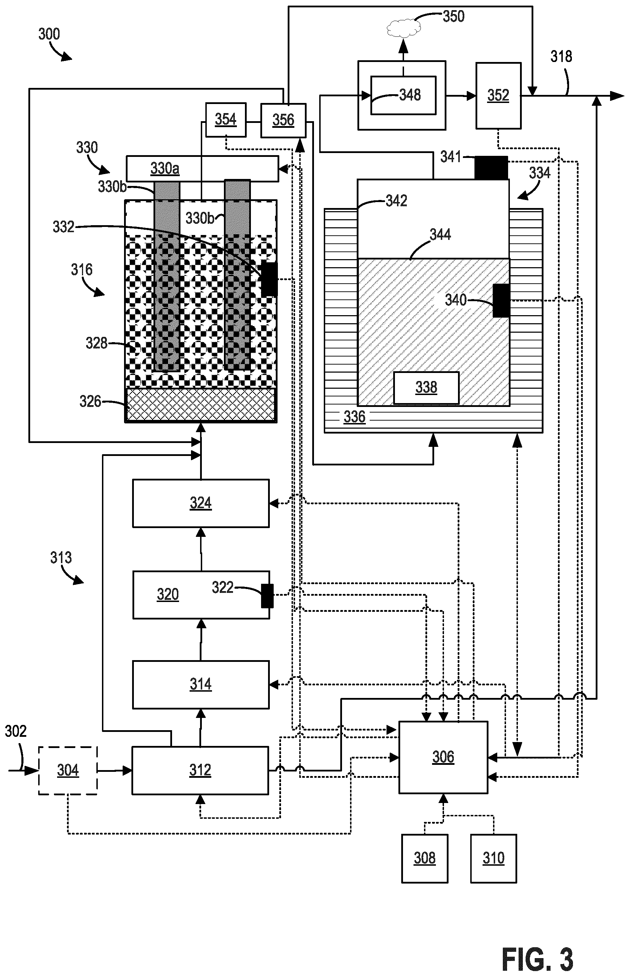

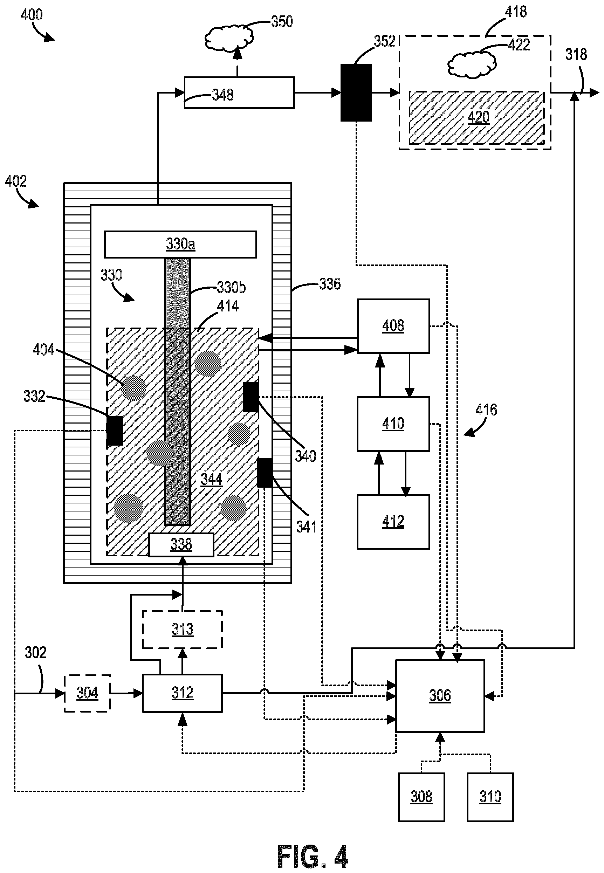

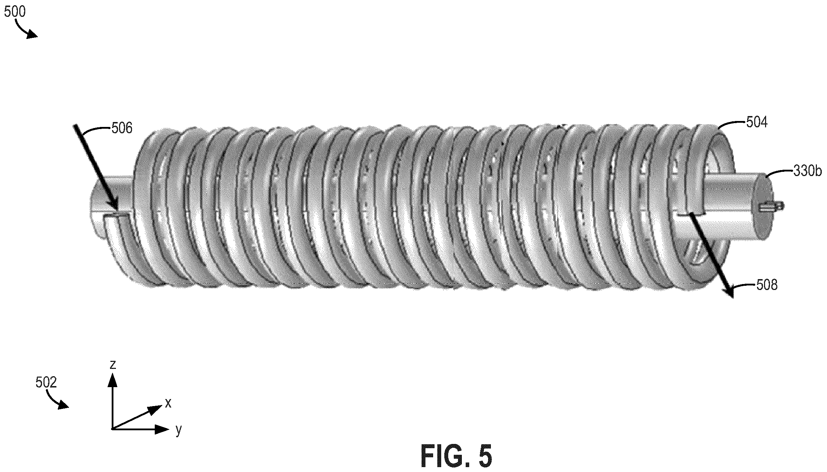

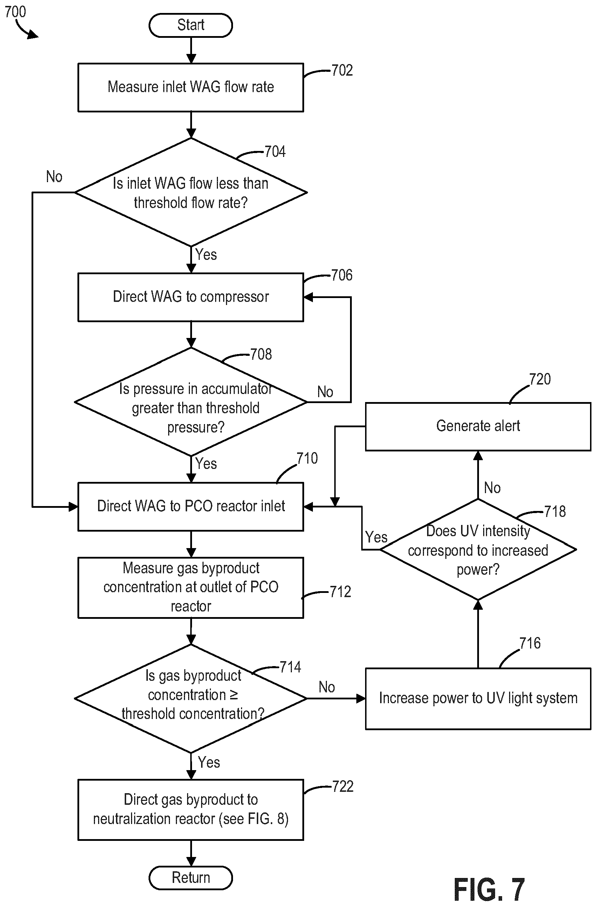

The following description relates to systems and methods for photocatalytic treatment of waste anesthetic gases. In an exemplary embodiment, the photocatalytic waste anesthetic gas (WAG) treatment system may be coupled to a closed loop anesthesia circuit as depicted in FIG. 1. To prevent collecting and venting the waste anesthetic gas to the environment, the photocatalytic WAG treatment system may chemically degrade the WAG into products which are not potent greenhouse gases and can thereafter be released to the environment. Herein, environment refers to atmosphere outside confines of a building (e.g., a hospital). The chemical degradation is referred to as photochemical oxidation and may be driven by superoxide and hydroxide radicals produced by interaction of ultraviolet (UV) light with a wide bandgap semiconductor oxide, such as titanium dioxide (TiO₂). The photochemical oxidation process is illustrated in FIG. 2. A first embodiment of the photocatalytic WAG treatment system is shown in FIG. 3. The photocatalytic waste anesthetic gas treatment system may include a system to compress and accumulate waste anesthetic gas output by the closed loop anesthesia circuit, thereby ensuring a desired gas flow rate into a photocatalytic oxidization (PCO) reactor. In some examples, the degradation of the waste anesthetic gas may result in acidic byproducts. The acidic byproducts may be directed to a neutralization reactor coupled to the photocatalytic waste anesthetic gas reactor before being output from the photocatalytic waste anesthetic gas treatment system. The photocatalytic WAG treatment system may further include sensors such as UV sensors, pH sensors, and chemical sensors, configured to monitor and direct the WAG and byproducts. In a second embodiment, shown in FIG. 4, the PCO reactor and neutralization reactor may be combined into a single hybrid reactor. The PCO reactor may be a packed bed reactor as depicted in FIG. 3 or a spiral flow reactor as depicted in FIGS. 5-6. In some examples, the PCO reactor may degrade waste anesthetic gas that does not produce acidic byproducts (e.g., N₂O) and may not demand coupling to a neutralization reactor. In alternate examples, the PCO reactor may be coupled to a neutralization reactor and may be used to treat waste anesthetic gas that does produce acidic byproducts. Methods for operating the photocatalytic WAG reactor, the neutralization reactor, and a liquid replacement system coupled to the neutralization reactor are shown in FIGS. 7-9.

Turning now to FIG. 1, an exemplary embodiment of a closed loop anesthesia circuit 100 coupled to a photocatalytic waste anesthetic gas treatment system 126 is shown. Fresh (e.g., not yet inhaled by a patient 107) anesthesia gas, from an anesthesia machine, may be introduced to the closed loop anesthesia circuit 100 at fresh gas inlet 102. Fresh anesthesia gas may flow through carbon dioxide absorber canister 104 and through inspiratory one-way valve 106 to patient 107. Inspiratory one-way valve 106 may be configured to allow gas flow towards the patient as indicated by arrow 108.

Additional compressed gas configured to drive a ventilator bellows 112 may enter closed loop anesthesia circuit 100 via compressed gas inlet 110. The ventilator bellows 112 may help to drive inhalation and exhalation by the patient 107, including inhalation of anesthetic gases. Gases exhaled by patient 107 may follow arrow 114 and flow through an expiratory one-way valve 116, through carbon dioxide absorber canister 104 and back through inspiratory one-way valve 106. Exhaled gases may be combination of carbon dioxide and waste anesthetic gases not absorbed through lungs of patient 107. Additionally, or alternatively, a reservoir bag 118 may be used to manually drive inhalation and exhalation by patient 107. A valve 120 may be configured to switch between closed loop anesthesia circuit 100 between being driven by ventilator bellow 112 and being driven by reservoir bag 118.

A ventilator exhaust valve 124 may be positioned in a flow path between ventilator bellows 112 and valve 120. When valve 120 is positioned to drive closed loop anesthesia circuit 100 by ventilator bellows 112, gas exhaled by patient 107 may exit closed loop anesthesia circuit 100 through ventilator exhaust via ventilator exhaust valve 124. Similarly, an adjustable pressure limiting (APL) valve 122 may be positioned in a flow path between reservoir bag 118 and valve 120. When valve 120 is position to drive closed loop anesthesia circuit 100 by reservoir bag 118, gas exhaled by patient 107 may exit closed loop anesthesia circuit 100 via APL valve 122.

Outlets of ventilator exhaust valve 124 and APL valve 122 may each be coupled to an inlet of a photocatalytic waste anesthetic gas treatment system 126. The photocatalytic waste anesthetic gas treatment system 126 may be configured to react waste anesthetic gases to benign components prior to disposal. In this way, release of excess anesthetic gases into the environment may be avoided. Additionally, photocatalytic waste anesthetic gas treatment system 126 may preclude a demand for onsite storage and subsequent transportation of excess anesthetic gas, thereby a reducing a time and cost associated with waste anesthetic gas treatment. It is understood that closed loop anesthesia circuit is an example of a system outputting waste anesthetic gas, but photocatalytic waste anesthetic gas treatment system 126 may be coupled to an output of other systems producing waste anesthetic gases.

A photocatalytic waste gas treatment system, such as photocatalytic waste anesthetic gas treatment system 126 may include a photocatalytic oxidizing reactor 128 comprising at least a source of UV light and an oxide catalyst. In examples where photocatalytic oxidation causes acidic gas byproducts, photocatalytic waste anesthetic gas treatment system 126 may additionally include a neutralization reactor 130. In some examples photocatalytic oxidizing 128 and naturalization reactor 130 may each be included in a hybrid photocatalytic oxidizing/neutralization reactor. The oxide catalyst may be wide bandgap semiconductor oxide configured to degrade waste anesthetic gases. In an exemplary embodiment the wide bandgap semiconductor oxide may be titanium dioxide (TiO₂). Other wide bandgap semiconductor oxides including, but not limited to, zinc oxide (ZnO) and cerium oxide (CeO₂) are considered within the scope of the disclosure.

Turning now to FIG. 2, the photocatalytic degradation of waste anesthetic gases by UV light in the presence of a wide bandgap semiconductor oxide is illustrated. UV light 202 may be directed to a wide bandgap semiconductor oxide 204. An electronic structure of wide bandgap semiconductor oxide 204 is shown schematically in illustration 200 of FIG. 2. The electronic structure includes a conduction band 206 and a valence band 208. An energy difference between a lowest electronic energy level of conduction band 206 and a highest electronic energy level of valence band 208 is a band gap (E_{g}) energy of the wide bandgap semiconductor oxide 204. UV light 202 may correspond to an energy equal to or greater than the band gap energy. For this reason, wide bandgap semiconductor oxide 204 may absorb UV light 202 and use the energy to promote an electron 216 from valence band 208 as indicated by arrow 210 to conduction band 206, leaving behind a hole 218 in the valence band 208.

Both the electron 216 and hole 218 may travel to a surface of wide bandgap semiconductor 204 as indicated by arrows 212 and 214 respectively. Electron 216 may react with oxygen as shown by arrow 212 to produce a superoxide radical (O₂^{·-}). Hole 218 may react with water to produce a hydroxide radical (^{·}OH). The superoxide radical and hydroxide radicals are highly energetic and may react readily with waste anesthetic gases forming carbon dioxide (CO₂), water (H₂O) and other byproduct gases. In some examples, the byproduct gases may include acidic byproduct gases such as hydrofluoric acid (HF) gas and hydrochloric acid (HCl) gas.

A wide bandgap semiconductor oxide and UV light may be incorporated to a photocatalytic waste anesthetic gas treatment system. A first embodiment of a photocatalytic waste anesthetic gas treatment system 300 is shown in FIG. 3. Solid lines connecting components of photocatalytic waste anesthetic gas treatment system 300 indicate fluid paths (e.g., fluid coupling) while dotted lines indicate communicative coupling. Communicative coupling of components may be wired or wireless. Photocatalytic waste anesthetic gas treatment system 300 may be an embodiment of photocatalytic waste anesthetic gas treatment system 126 of FIG. 1. In some examples, waste anesthetic gas treatment system 300 may be coupled to an outlet of a closed loop anesthesia circuit, as shown in FIG. 1.

Waste anesthetic gas may enter photocatalytic waste anesthetic gas treatment system 300 via waste anesthetic gas inlet 302. In some examples a flow sensor 304 may optionally be positioned in a flow path of the waste anesthetic gas downstream of waste anesthetic gas inlet 302. The flow sensor 304 may be configured to sense a flow rate of the waste anesthetic gas entering waste anesthetic gas treatment system 300. The flow sensor 304 may be communicatively coupled to a controller 306. In alternate examples, controller 306 may be communicatively coupled to a flow sensor of an anesthesia circuit (e.g., closed loop anesthesia circuit 100) fluidly coupled to photocatalytic waste anesthetic gas treatment system 300, configured to communicate a flow rate of waste anesthetic gas into photocatalytic waste anesthetic gas treatment system 300.

Controller 306 may receive input data from the various sensors described herein, process the input data, and trigger various actuators in response to the processed input data based on executable instruction or code programmed therein corresponding to one or more routines. Controller 306 may include non-volatile memory configured to store instructions and a processor configured to execute instructions. Controller 306 may be further coupled to a display 308 and a user interface 310. In some examples, user interface 310 and display 308 may be a mobile device communicably coupled to controller 306.

Waste anesthetic gas may flow to inlet gas diversion valve 312. Inlet gas diversion valve 312 may be configured to direct waste anesthetic gas to a gas accumulator 313, to a photocatalytic oxidizing reactor 316, or an outlet 318 of photocatalytic waste anesthetic gas treatment system 300. Inlet gas diversion valve 312 may be communicatively coupled to controller 306 and may receive instructions to divert gas according to input of sensors coupled to controller 306. For example, in response to a sensed flow rate waste anesthetic gas downstream of inlet gas diversion valve 312 (e.g., from flow sensor 304) below a threshold flow rate, inlet gas diversion valve 312 may be configured to direct waste anesthetic gas to gas accumulator 313. The threshold flow rate may be a flow rate below which waste anesthetic gas may not be effectively or evenly distributed through photocatalytic oxidizing reactor 316. As one example, the threshold flow rate may be 2.0 standard liters per minute (slpm). In response to the sensed flow rate above the threshold flow rate, inlet gas diversion valve 312 may be configured to direct waste anesthetic gas directly to photocatalytic oxidizing reactor 316, thereby bypassing compressor 314.

Gas accumulator 313 may include a compressor 314 fluidly coupled to an accumulator tank 320. Accumulator tank 320 may be a receptacle, such as a rigid container, configured to receive the waste anesthetic gas output by compressor 314. Accumulator tank 320 may include a pressure sensor 322 communicatively coupled to controller 306 and configured to output a gas pressure inside accumulator tank 320. An accumulator output valve 324 may be positioned downstream of accumulator tank 320 and in a fluid path between accumulator tank 320 and photocatalytic oxidizing reactor 316. Accumulator output valve 324 may be communicatively coupled to controller 306. In one example, controller 306 may adjust accumulator output valve 324 from a closed position to an open position in response to a pressure in accumulator tank 320, as reported by pressure sensor 322, being above the threshold pressure. In this way, accumulator tank 320 may store waste anesthetic gas and build up pressure when a flow rate of waste anesthetic gas input into photocatalytic waste anesthetic gas treatment system 300 is below the threshold flow rate.

Waste anesthetic gases may be directed to photocatalytic oxidizing reactor 316 where photocatalytic decomposition of waste anesthetic cases occurs, via photocatalytic oxidation as described above with respect to FIG. 2. Photocatalytic oxidizing reactor 316 may include a distributor 326, oxide coated porous ceramic pellets 328 coated with oxide catalyst, such as TiO₂, a UV light system 330 and an ultraviolet sensor 332. In one example, photocatalytic oxidizing reactor 316 may be a packed bed photocatalytic oxidizing reactor.

Distributor 326 may be directly coupled to an inlet of PCO reactor 316 and may be configured to evenly distribute waste anesthetic gas throughout a volume of photocatalytic oxidizing reactor 316. In this way, tunneling of waste anesthetic gas through photocatalytic oxidizing reactor 316 may be prevented. In some examples distributor 326 may be a glass frit.

Oxide coated porous ceramic pellets 328 may be uniformly coated with wide bandgap semiconductor oxide. Pores of oxide coated porous ceramic pellets 328 may be configured to increase an exposed surface area and the oxide coating of oxide coated porous ceramic pellets 328 includes coating on an interior surface of the pores. In this way, waste anesthetic gas may pass across surfaces and through pores of porous ceramic pellets and react with the free radical species (e.g., superoxide and hydroxide radicals) generated by reaction of the wide bandgap semiconductor oxide with UV light emitted by UV light system 330.

UV light system 330 may include a power source 330a and UV lamp 330b. In one example UV light system 330 may include two UV lamp 330b, however other numbers of UV lamp 330b have also been considered within a scope of this disclosure. In one example UV lamp 330b may be mercury lamp. In alternate examples UV lamp 330b may be a light emitting diode (LED). A range of wavelengths emitted by UV light system 300 may be chosen based on a band gap energy of the wide bandgap semiconductor oxide. For example, if the wide bandgap semiconductor oxide is TiO₂ than the UV lamp may be selected to emit wavelengths of at least 380 nm or lower (e.g., higher energy). In some examples the range of wavelengths may be between 260 nm and 350 nm. UV lamp 330b may be positioned inside a housing of PCO reactor 316. UV lamp 330b may configured to excite the wide bandgap semiconductor oxide coating of the oxide coated porous ceramic pellets 328. Power source 330a may be communicatively coupled to controller 306. UV sensor 332 may be configured to output a signal proportional to an intensity of UV light emitted by UV light system 330. In response to a UV light intensity below a threshold intensity, controller 306 may include instructions to increase a power output of power source 330a to increase UV light intensity. If the power output of power source 330a is already at a maximum power output and the UV light intensity is below the threshold intensity, controller 306 may include instructions to output a visual or auditory alert to prompt a user to replace UV lamp 330b. In one example a visual alert may be displayed at display 308. In addition to displaying an alert, controller 306 may actuate inlet gas diversion valve 312 to direct waste anesthetic gas directly to outlet 318. In this way, breakthrough waste anesthetic gas if UV lamp 330b demands replacement may be avoided.

An output of photocatalytic oxidizing reactor 316 may be directed to an inlet of a byproduct chemical sensor 354. The output of photocatalytic oxidizing reactor 316 may be decomposition gas byproducts of anesthetic gas degradation by free radical species. Decomposition gas byproducts may include acidic gas byproducts including hydrogen fluoride and hydrogen chloride gases in addition to other gases. Byproduct chemical sensor 354 may be communicatively coupled to controller 306 and configured to determine a concentration of chemical byproducts output by photocatalytic oxidizing reactor 316. In one example byproduct chemical sensor 354 may include an array of chemical sensors, each configured to selectively, quantitatively detect a chemical component, or class of chemical components of decomposition gas byproducts. In one example, byproduct chemical sensor 354 may be an infrared sensor.

A diversion valve 356 may be positioned downstream of byproduct chemical sensor 354 and upstream of a neutralization reactor 334. Diversion valve 356 may be communicatively coupled to controller 306. In response to byproduct chemical sensor 354 measuring a concentration of decomposition gas byproducts below a threshold concentration, controller 306 may adjust diversion valve 356 to direct gas output from PCO reactor 316 back to an input of PCO reactor 316. The threshold concentration may be determined by the controller based on a known composition of the waste anesthetic gas as well as the threshold flow rate and/or threshold pressure. In response to byproduct chemical sensor 354 measuring a concentration of decomposition gas byproducts greater than or equal to the threshold concentration, controller 306 may adjust diversion valve 356 to direct gas output from PCO reactor 316 to neutralization reactor 334. In some examples, controller 306 may also adjust diversion valve 356 to bypass neutralization reactor 334 and direct an output of PCO reactor 316 to outlet 318.

Hydrogen fluoride and hydrogen chloride are acidic, corrosive gases that may be included in decomposition gas byproducts. For this reason, the neutralization reactor 334 is configured to neutralize acidic gases (e.g., acidic species) such as hydrogen fluoride and hydrogen chloride to prevent exposure of the acidic gases to people, non-acid resistant equipment, and the environment.

Neutralization reactor 334 may include a cooling system 336, a distribution system 338, a pH sensor 340, and a replaceable cartridge 342. Replaceable cartridge 342 may include a neutralization solution 344. In some examples, a temperature sensor 341 may be in face sharing contact with an outer surface of replaceable cartridge 342 and configured to determine a temperature of the cartridge housing. Before operation of photocatalytic waste anesthetic gas treatment system 300, neutralization solution 344 may be an aqueous basic solution. As one example, neutralization solution 344 may be solution of one or more weak bases such as calcium hydroxide or sodium bicarbonate. Gaseous byproducts from photocatalytic oxidizing reactor 316 may be directed to neutralization solution 344 via distribution system 338. Distribution system 338 may include a sparging filter and/or magnetic stirrer positioned at an inlet of replaceable cartridge 342 and configured to efficiently dissolve the gaseous byproducts into neutralization solution 344.

Upon dissolving into neutralization solution 344, the acidic gaseous byproducts (e.g., HF and HCl gas) may react with the basic species of the neutralization solution 344 to produce water and a neutral salt. In some examples, the neutral salt may not be soluble in water and may accumulate as a precipitate within replaceable cartridge 342. Over time, the acidic gaseous byproducts may use up the basic species and a pH of neutralization solution 344 may decrease from a basic pH towards a neutral/acidic pH. For this reason, pH sensor 340 may be communicatively coupled to controller 306 and may be configured to monitor a pH of neutralization solution 344. In response to a pH lower than a threshold pH, controller 306 may alert a user (e.g., via display 308) to replace replaceable cartridge 342 with a new cartridge. The threshold pH may be greater than 7.0. In one example the threshold pH may be equal to 7.5. The new cartridge may include a fresh basic solution at a basic pH. In some examples, in response to a pH lower than a neutral pH (e.g., pH 7.0), controller 306 may actuate inlet gas diversion valve 312 to direct waste anesthetic gas directly to outlet 318. In one example, outlet 318 may be fluidly coupled to an anesthetic gas scavenging system. The anesthetic gas scavenging system may capture or direct gases to be released outside to the environment (e.g., to the environment outside a hospital building). In this way, the photocatalytic reaction producing the acidic byproducts may be avoided when neutralization reactor 334 is not able to neutralize the acidic byproducts. In some examples the anesthetic gas scavenging system may include a vacuum configured to place the anesthetic gas scavenging system and photocatalytic waste anesthetic gas treatment system 300 under negative pressure.

The neutralization reaction between acidic gaseous byproducts and basic species may be an exothermic reaction. For this reason, cooling system 336 may include a jacket circumferentially surround replaceable cartridge 342. In one example, cooling system 336 may be a solid state cooling system such as a piezoelectric cooler with a cold side in face sharing contact with replaceable cartridge 342. In an alternate example, cooling system 336 may be fluidly coupled to a circulating chiller configured to output cold fluid to an inlet of the cooling jacket and receive warm fluid from an outlet of the cooling jacket. In a further embodiment, cooling system 336 may be an immersion cooling system positioned in direct face sharing contact with fluid in the cooling jacket or in direct face sharing contact with neutralization solution 344. In some examples, cooling system 336 may include a temperature sensor configured to monitor a temperature of the coolant. Cooling system 336 may be communicatively coupled to controller 306. Controller 306 may controller a target temperature of cooling system 336.

An output of neutralization reactor 334 may include non-acidic gases, including water vapor and in some examples, CO₂ may be produced as a result of the neutralization reaction. In some examples, at least a portion of a fluid path 348 of the output of neutralization reactor 334 may be formed of a selectively permeable material. The selectively permeable material may be configured to allow water vapor 350 to readily pass through the material while other gases such as oxygen and carbon dioxide are retained within. As one example, the selectively permeable material may be a sulfonated tetrafluoroethylene based fluoropolymer. In some examples, a waste heat from cooling system 336 may be direct towards fluid path 348. In this way a relative humidity of air surrounding fluid path 348 may be decreased and a driving force (e.g., a rate) of water vapor 350 out of fluid path 348 may be increased.

A chemical sensor 352 may be positioned downstream of neutralization reactor 334 and upstream of outlet 318. Chemical sensor 352 may be configured to sense levels (e.g., concentrations) of breakthrough gas flowing out of neutralization reactor 334. Breakthrough gas may include breakthrough acidic gases (e.g., HF and HCl) and/or breakthrough waste anesthetic gases present in gas output from neutralization reactor 334. In one example chemical sensor 352 may be an array of chemical sensors, each configured to sense a gas of interest. In some examples, chemical sensor 352 may include one or more infrared (IR) sensors. In alternate examples, chemical sensor 352 may include one or more of a photoionization sensor, an electrochemical sensor, or other types of sensors configured to selectively and quantitatively monitor presence of hazardous gases. Chemical sensor 352 may be communicatively coupled to controller 306. Controller 306 may include instructions to generate an alert if levels of acidic gases and/or waste anesthetic gases are above a threshold level. Additionally, controller 306 may actuate inlet gas diversion valve 312 to direct waste anesthetic gas directly to outlet 318 in response to levels of acidic gases and/or waste anesthetic gases above a threshold level.

Turning now to FIG. 4, an alternate embodiment of a photocatalytic waste anesthetic gas treatment system 400 is shown. Photocatalytic waste anesthetic gas treatment system 400 may include a hybrid PCO/neutralization reactor 402 in place of PCO reactor 316 and neutralization reactor 334 of FIG. 3. Alternate embodiment 400 may include similar components as photocatalytic waste anesthetic gas treatment system 300. Similar components are numbered the same and may not be reintroduced.

In some examples, waste anesthetic gas treatment system 400 may include an inlet gas diversion valve 312 and optionally a gas accumulator 313, similar to waste anesthetic gas treatment system 300. In alternate examples, hybrid PCO/neutralization 402 reactor may be configured to react effectively for a wide range of input gas flow rates. In such examples, waste anesthetic gas treatment system 400 may not include gas accumulator 313. Accordingly, in such examples, inlet gas diversion valve 312 may be configured to direct waste anesthetic gas to hybrid PCO/neutralization reactor 402 or to outlet 318.

Waste anesthetic gas may be directed into a reaction cartridge 414 via distribution system 338. Reaction cartridge 414 may include neutralization solution 344 and suspended wide bandgap semiconductor particles 404. In one example wide bandgap semiconductor oxide particles may be in a size range between 12 nm and 29 nm in diameter. In some examples, a diameter of the wide bandgap semiconductor oxide particles may be at most 29 nm. In some embodiments, the wide bandgap semiconductor oxide particles may be roughly spherical, although other shapes of particles such as rods or ellipsoids are also considered. The rate of photocatalysis may be inversely dependent on a size of the wide bandgap semiconductor oxide. As a size of the wide bandgap semiconductor oxide particle increases, a photocatalytic rate constant may decrease exponentially. Free radicals may be generated by excitation of wide bandgap semiconductor particles 404 with UV light from UV lamp 330b and may react with waste anesthetic gases to degrade waste anesthetic gases as described above with respect to FIG. 2. Additionally, components of waste anesthetic gas may degrade via direct reaction with the basic species of neutralization solution 344. In an example where the basic species is sodium bicarbonate and the waste anesthetic gas includes compound A (CF₂=C(CF₃)-O-CH₂F), compound A may not be soluble in the sodium bicarbonate solution and may therefore form a precipitate. In an alternate example where the basic species is sodium bicarbonate and the waste anesthetic gas includes pentafluoropropanol and pentafluoropropanol may react exothermically with sodium bicarbonate to form trifluoromethyl acetate and carbon dioxide. Neutralization reaction may also occur in neutralization solution 344 as acidic byproducts may be reacted with basic species directly after forming near a surface of wide bandgap semiconductor particles 404.

In some examples, neutralization solution 344 may demand replacing before replacement of UV light system 330. In such an example, a liquid replacement system 416 may be fluidly coupled to reaction cartridge 414. In some examples, liquid replacement system 416 may be fluidly coupled to a PCO reactor, such as PCO reactor 316 of FIG. 3. Liquid replacement system 416 may include a self-sealing fluid cartridge 412, a bidirectional pump 410 and a liquid shutoff valve 408. Self-sealing fluid cartridge 412 may include a fresh neutralization solution. A volume of fresh neutralization solution included in self-sealing fluid cartridge 412 may be equivalent to a volume of neutralization 344 included in reaction cartridge 414. In some examples, fresh neutralization solution may include replacement wide bandgap semiconductor particles 404. In alternate examples, liquid shutoff valve may be configured to selectively pass aqueous solution while forcing wide bandgap semiconductor particles to remain in reaction cartridge 414. In such examples, the fresh neutralization solution may not include wide bandgap semiconductor particles 404. In some examples, self-sealing fluid cartridge 412 may also include an empty volume configured to receive spent neutralization solution. Self-sealing fluid cartridge 412 may be fluidly coupled to bidirectional pump 410. Liquid shutoff valve 408 may be positioned in a fluid path between bidirectional pump 410 and reaction cartridge 414 and may be configured to control flow of liquid to and from reaction cartridge 414. Liquid shutoff valve 408 and bidirectional pump 410 may each be communicatively coupled to controller 306. Controller 306 may include instructions to actuate bidirectional pump and liquid shutoff valve 408 to flow spent neutralization solution from reaction cartridge 414 to an empty volume of self-sealing fluid cartridge 412 and to actuate bidirectional pump 410 to and liquid shutoff valve 408 to flow fresh neutralization from self-sealing fluid cartridge 412 into reaction cartridge 414. When full of spent neutralization solution and empty of fresh neutralization, self-sealing fluid cartridge 412 may be configured to be removed from liquid replacement system 416 for disposal.

In some examples, a condensation trap 418 may be positioned downstream of chemical sensor 352 and upstream of outlet 318. A fluid path of condensation trap 418 may be cooled to a temperature of -80 °C or lower. In some examples a temperature of condensation trap 418 may be maintained above -189°C to prevent condensation of O₂. In this way, at least some of the fluorinated compounds not degraded to HF by photocatalytic oxidation reactor may be captured in liquid trap 420 for later disposal. In one example the fluorinated compound may be fluoromethyl-2, 2-difluoro-1-(hydroxymethylpropane). Remaining gases 422, such as CO₂ and O₂ may continue through outlet 318. In some examples condensation trap 418 may be included in waste anesthetic gas treatment system 300 of FIG. 3 and positioned downstream of chemical sensor 352 and upstream of outlet 318.

In some examples, where high throughput is demanded, an alternate embodiment 500 of a photocatalytic oxidizing reactor may be included in a photocatalytic waste anesthetic gas treatment system, such as to replace the photocatalytic oxidizing reactor 316 of photocatalytic waste anesthetic gas treatment system 300. In some examples, alternate embodiment 500 may be configured to treat non-halogenated waste anesthetic gas, such as nitrous oxide. In such an example, byproducts of waste anesthetic gas oxidation may not be acidic and a neutralization reactor may not be included in the photocatalytic waste anesthetic gas treatment system.

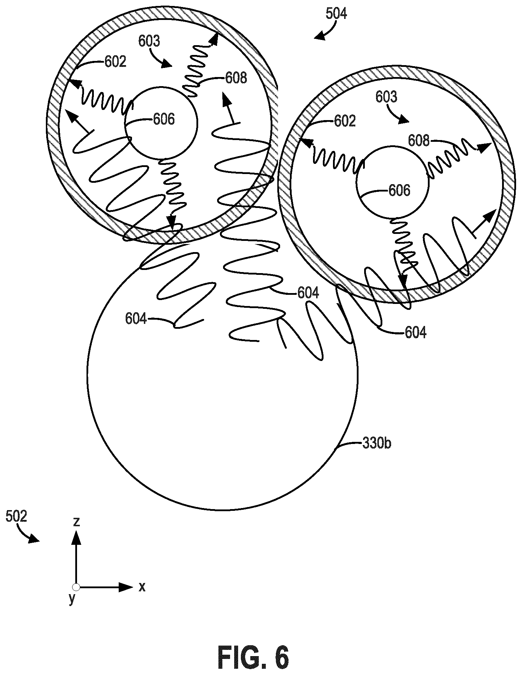

Alternate embodiment 500 is shown in FIG. 5 and FIG. 6. FIG. 5 shows an illustration of a side view of the alternate embodiment 500 and FIG. 6 shows a cross sectional view of the alternate embodiment 500. A reference axis 502, including an x-axis, y-axis, and z-axis is provided for comparison between FIGS. 5 and 6. FIG. 5 and FIG. 6 may include similar components as photocatalytic waste anesthetic gas treatment system 300 and 400 of FIGS. 3 and 4 respectively. Similar components will be labeled the same and may not be reintroduced.

The alternate embodiment 500 of a photocatalytic oxidizing reactor may include a UV lamp 330b. In one example UV lamp 330b may be shaped as a cylinder. The y-axis may be an axial axis with respect to UV lamp 330b. An ultraviolet transparent tube 504 may be placed in face sharing contact with UV lamp 330b or a threshold distance from face sharing contact from an outer surface of UV lamp 330b. UV transparent tube 504 may be a hollow tube including an interior lumen, and waste anesthetic gas may flow through the lumen of UV transparent tube 504 through a first end as indicated by arrow 506 and out of a second end, the second end distal from the first end, as indicated by arrow 508. UV transparent tube 504 may be formed of a UV transparent material. For example, UV transparent tube 504 may be formed of quartz. In one example, UV transparent tube 504 may include a single lumen wound around counter-axially around an axial length of UV lamp 330b.

A lumen 603 of UV transparent tube 504 is shown in more detail in the cross sectional view of FIG. 6. FIG. 6 shows an alternate embodiment of UV transparent tube 504, including a plurality of UV transparent tubes 504 arranged co-axially with UV lamp 330b. In some examples, UV transparent tubes may be arranged co-axially and spirally wound about UV lamp 330b along the y-axis. A cross-section of UV transparent tube 504 may be similar in both the co-axial arrangement of FIG. 6 and the counter-axial and helical arrangement of FIG. 5.

An inner surface 602 of UV transparent tube 504 may be coated with a wide bandgap semiconductor oxide (e.g., TiO₂). In this way, UV light 604 may radiate from UV lamp 330b through UV transparent tube 504 and excite the wide bandgap semiconductor coating, thereby emitting free radical species to react with and degrade waste anesthetic gases. In some examples, silver particles may be deposited onto the wide bandgap semiconductor coating. The silver particles may enhance electron/hole separation, thereby improving an efficiency of free radical generation by the wide bandgap semiconductor. Additionally, an ultraviolet transmitting fiber optic 606 may be positioned concentrically within lumen 603 of UV transparent tube 504. In some examples a first axial end of UV transmitting fiber optic 606 may be coupled to an additional UV light source, such as a UV LED. UV transmitting fiber optic 606 may be configured to both pass UV light from the first axial end of UV transmitting fiber optic 606 to a second axial end of UV transmitting fiber optic 606 by total internal reflection. Additionally, UV transmitting fiber optic 606 may be configured to reflect UV light 608 out of UV transmitting fiber optic 606 towards inner surface 602 of UV transparent tube 504. Additionally or alternatively, UV transmitting fiber optic 606 may reflect UV light emitted by UV lamp 330b. In this way, interaction between UV light and wide bandgap semiconductor coated inner surface 602 may be increased, thereby increasing a rate of formation and concentration of free radicals and increasing a subsequent rate of photocatalytic degradation of waste anesthetic gas.

Turning now to FIG. 7 a flow chart of a method 700 for operating a photocatalytic oxidation reactor of a photocatalytic waste anesthetic gas treatment system is shown. Instructions for carrying out method 700 and the rest of the methods included herein may be executed by a controller (e.g., controller 306) based on executable instructions stored on a non-volatile memory of the controller and in conjunction with signals received from sensors of the photocatalytic waste anesthetic gas system, such as the sensors described above with reference to FIG. 3. The controller may employ actuators of the photocatalytic waste anesthetic gas system to adjust flow of the waste anesthetic gases, according to the methods described below. The photocatalytic oxidation reactor of method 700 may a packed bed reactor such as photocatalytic oxidizing reactor 316 of FIG. 3, hybrid PCO/neutralization reactor of FIG. 4 or a spiral flow reactor such as alternate embodiment 500 shown in FIGS. 5 and 6.

At 702, method 700 includes measuring an inlet waste anesthetic gas (WAG) flow rate. In some examples, the WAG flow rate may be measured by an inlet flow sensor, such as inlet flow sensor 304 of FIG. 3. In alternate examples, the controller may be communicatively coupled to an anesthesia system fluidly coupled to an inlet of the waste anesthetic gas treatment system and measuring WAG flow rate may include receiving a flow rate from a sensor of the anesthesia system.

At 704, method 700 includes determining if the inlet WAG flow rate is less than a threshold flow rate. The threshold flow rate may be a flow rate below which waste anesthetic gas may not be effectively treated by the photocatalytic oxidization reactor (PCO). The threshold flow rate may be based on a configuration of a the PCO reactor. For example, a PCO reactor configured as a packed bed reactor (e.g., PCO reactor 316 of FIG. 3) may have a different threshold flow rate than hybrid PCO/neutralization reactor 402 or flow through PCO reactor 500. In some examples, the threshold flow rate may be dependent on a desired backpressure for operation of the photocatalytic oxidization reactor.

If at 704, method 700 determines that the inlet WAG flow rate is less than the threshold flow rate (YES) than method 700 continues to 706 and includes directing WAG to a compressor. The compressor may be similar to compressor 314 of FIG. 3. The compressor may force the WAG into an accumulator tank, such as accumulator tank 320 of FIG. 3. Method 700 then continues to 708 and determines if a pressure in the accumulator tank is greater than a threshold pressure. The pressure in the accumulator tank may be determined by a pressure sensor fluidly coupled to the accumulator and communicatively coupled to the controller. The threshold pressure may be a pressure associated with ability to flow WAG from the accumulator tank to the PCO reactor at a flow rate above the threshold flow rate. If the pressure in the accumulator is not greater than the threshold pressure (NO), method 700 returns to 706 and continues to direct WAG to the compressor. If the pressure is greater than the threshold pressure (YES), method 700 continues to 710 and includes directing WAG to an inlet of the PCO reactor. If at 704, method 700 determines that the inlet WAG flow rate is greater than or equal to the threshold flow rate (NO) than method 700 also continues to 710.

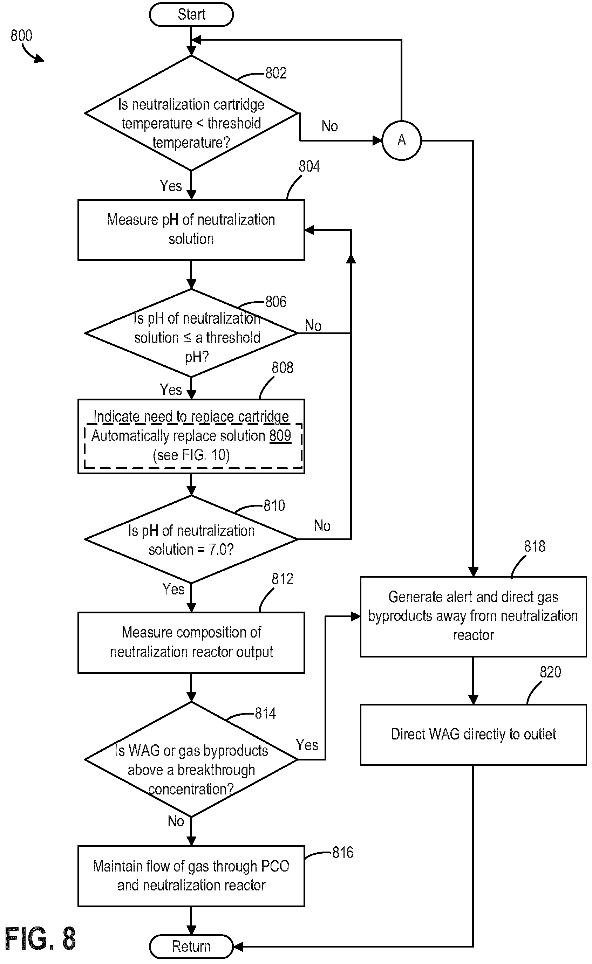

At 712, method 700 includes measuring gas byproduct concentration at an outlet of the PCO reactor. The gas product may be measured by a chemical sensor positioned at an outlet of the PCO reactor. At 714, method 700 determines if the measured concentration of gas byproduct is greater than or equal to a threshold concentration. The threshold concentration may correspond to an expected concentration of gas byproducts based on the WAG input and assuming substantially total (e.g., within 5%) degradation of the WAG. The threshold concentration may be determined by the controller based on the threshold flow rate and the threshold pressure. If at 714, method 700 determines that the gas byproduct is greater than or equal to the threshold concentration (YES), method 700 proceeds to 722 and includes directing gas byproducts to a neutralization reactor. Methods for flowing gas byproducts through the neutralization reactor are described further below with respect to FIG. 8.

If at 714, method 700 determines that the measured concentration of gas byproduct is less than the threshold concentration (NO), method 700 proceeds to 716 and includes increasing power (e.g., operating power) to a UV light system of the PCO reactor. The UV light system may be similar to UV light system 330 of FIGS. 3 and 4. Increasing power of the UV light system may increase a number of photons emitted from a UV lamp of the UV light system. Method 700 continues to 718 and determines if an in increase in UV intensity in the PCO reactor corresponding to the increase in UV power is observed. UV intensity in the PCO reactor may be determined by a UV sensor positioned within the PCO reactor and communicatively coupled to the controller. If at 718, method 700 determines the UV intensity does correspond to increased power (YES), method 700 returns to 710 and directs gas from an outlet of the PCO reactor back to an inlet of the PCO reactor. If at 718, method 700 determines the UV intensity does not correspond to increased power (NO) method 700 proceeds to 720 and includes generating an alert. The alert may be generated at a display communicatively coupled to the controller. Additionally or alternatively, the alert may include an audio signal such as an alarm or chime. The alert may prompt a user to direct WAG to an outlet of the waste anesthetic gas treatment system and to replace the UV lamp. Additionally or alternatively, the alert may prompt a user to adjust the threshold flow rate and the threshold pressure. Method 700 may proceed to 710 and continue to recycle WAG back to an inlet of the PCO reactor. Method 700 may proceed in this manner until the gas byproduct concentration determined at step 714 is greater than or equal to the threshold concentration and gas byproducts are directed to the neutralization reactor at 722. Method 700 returns.

Turning now to FIG. 8, a flow chart of a method 800 for operating a neutralization reactor a waste anesthetic gas treatment system, such as waste anesthetic gas treatment system 300 of FIG. 3 is shown. In some examples, method 800 may continue from method 700 after method 700 determines that sufficient degradation of waste anesthetic gases at an output of the PCO reactor. In some examples, method 800 may be optional and may be omitted if degradation of waste anesthetic gas is not expected to produce acidic gas byproducts. In some examples the neutralization reactor may be a hybrid PCO/neutralization reactor, such as hybrid PCO/neutralization reactor 402 of FIG. 4.

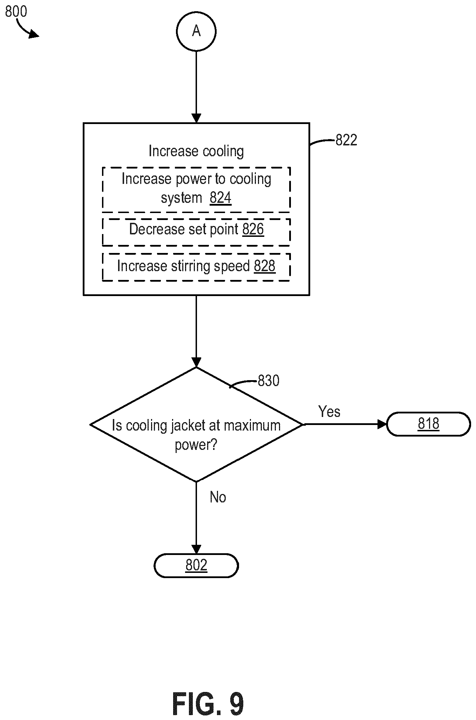

At 802, method 800 determines if a temperature of the neutralization cartridge is less than a threshold temperature. The temperature of the neutralization cartridge may be determined by a temperature sensor in face sharing contact with an outer surface of the neutralization cartridge and communicatively coupled to the controller. The neutralization cartridge may be a replaceable cartridge (e.g., replaceable cartridge 342 of FIG. 2) or cartridge 414 of FIG. 4. The threshold temperature may be a temperature above which degradation of components of the neutralization system may occur. In one example, the threshold temperature may be 50 °C. If method 800 determines that the cartridge housing temperature is greater than the threshold temperature (NO), method 800 continues to 822 shown in FIG. 9 and includes increasing cooling. Increasing cooling may include increasing a power used by the cooling system to cool the neutralization cartridge. Increasing cooling may include, at 824, increasing power to a cooling system (e.g., cooling system 336 of FIG. 3). Additionally, in some examples, increasing cooling may include, at 826, decreasing set-point temperature to of the cooling system to a colder temperature. Further, increasing cooling may include, at 828, increasing a stirring speed of a distribution system of the neutralization reactor. Increasing stirring speed may increase contact between a bulk of the neutralization solution and cold elements of the cooling system, thereby increasing cooling. Method 800 continues to 830 and determines if the cooling system is at maximum power. In some examples, the cooling system may be at maximum power if the cooling system is set to a coldest possible temperature. If at 830, method 800 determines that the cooling system is not at the maximum power (NO), method 800 returns to 802 shown in FIG. 8 and again determines if the temperature of the neutralization cartridge is less than the threshold temperature. If at 830, method 800 determines that the cooling system is at the maximum power (YES), method 800 continues to 818, shown in FIG. 8, and includes directing gas byproducts away from the neutralization as described further below.

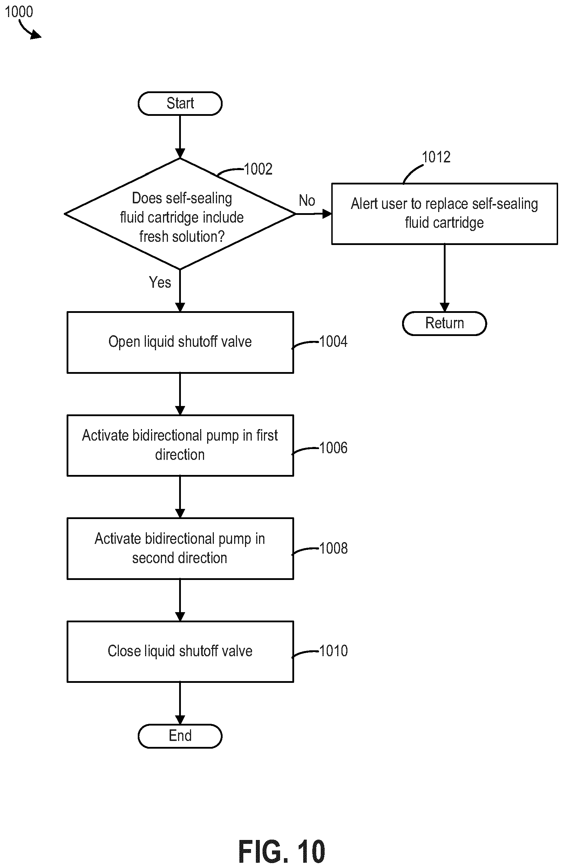

If at 802, method 800 determines that the neutralization cartridge temperature is less than the threshold temperature (YES), method 800 proceeds to 804 and includes measuring pH of the neutralization solution. The pH of the neutralized solution may be measured using a pH sensor positioned within the neutralization solution and communicatively coupled to the controller. As the neutralization system operates, the pH of the neutralization increases as basic species of the neutralization solution are used up. In some examples, pH of the neutralization solution may be continuously monitored. At 806, method 800 determines if the pH (e.g., the monitored pH) of the neutralization solution is less than or equal to a threshold pH. The threshold pH may be above pH 7.0. In one example, the threshold pH may be 7.5. If method 800 determines that the pH of the neutralization solution not less than or equal to the threshold pH (NO), method 800 returns to 804 and continues to monitor pH of the solution. If method 800 determines that the pH of the neutralization solution is less than or equal to the threshold pH (YES), method 800 proceeds to 808 and includes instructing the user replace the cartridge (e.g., neutralization cartridge) soon. Instructing the user to replace the neutralization cartridge soon may include generating an audio and/or visual alert. In one example the visual alert may be generated at the display. In some examples, at 809, method 800 includes automatically replacing neutralization solution in the cartridge. A liquid replacement system, such as liquid replacement system 416 of FIG. 4 may be configured to automatically replace the used (e.g., pH ≤ 7.5) neutralization solution with fresh neutralization solution. Operation of the liquid replacement system is described further below with respect to FIG. 10.

At 810, method 800 determines if pH of neutralization solution is equal to 7.0. If pH of the neutralization solution is not equal to 7.0 (NO), method 800 returns to 804 and continues to measure pH of the neutralization solution. If pH of the neutralization solution is equal to 7.0 (YES), method 800 continues to 812 and includes measuring a composition of neutralization reactor output. Composition of neutralization reactor output may be measured by a chemical sensor positioned downstream of an outlet of the neutralization reactor and upstream of an outlet of the waste anesthetic gas treatment system (e.g., chemical sensor 352). The chemical sensor may be configured to detect concentration of acidic byproduct gases (e.g., HF and HCl) as well as concentration of waste anesthetic gases.

At 814, method 800 determines if WAG or acid gas byproducts are above a breakthrough concentration. In some examples, a breakthrough concentration may be a value greater than zero that corresponds to a lowest sensitivity of the chemical sensor. Said another way, the breakthrough concentration may be a lowest concentration reported by the chemical sensor that is believed to be a real, reliable measurement. In alternate examples, the breakthrough concentration be equivalent to a threshold exposure limit. If method 800 determines that WAG or acid gas byproducts are not above the breakthrough concentration (NO), method 800 proceeds to 816 and includes maintaining a flow of WAG to the PCO and gas byproducts from the PCO to the neutralization reactor. Method 800 returns.

If method 800 determines that WAG or acid gas byproducts are above the breakthrough concentration, method 800 proceeds to 818 and includes generating an alert and directing gas byproducts products away from the neutralization reactor and the outlet of the waste anesthetic gas treatment system. Gas byproducts may be directed by actuating a valve positioned in a flow path between the outlet of the PCO and the neutralization reactor. Generating an alert may include generating an audio or visual alert. The visual alert may be shown at the display. The alert may convey to the user the breakthrough is occurring and that WAG and byproduct gases are being directed to the outlet. At 820, method 800 includes directing WAG directly to the outlet of the photocatalytic waste anesthetic gas treatment system. Directing WAG directly to the outlet of the photocatalytic waste anesthetic gas may include actuating an inlet gas valve positioned upstream of the PCO reactor. In this way, WAG and acid gas byproducts may be directed outside of an enclosed area (e.g., operating room) and stored or vented to the environment when there is a danger that WAG or acid gas byproducts may vent from the waste anesthetic gas treatment system into the enclosed area. Method 800 returns.

Turning now to FIG. 10, a flow chart of a method 1000 for automatically replenishing a neutralization solution of a photocatalytic waste anesthetic gas treatment system is shown. The neutralization solution may be automatically replenished in examples where the photocatalytic waste anesthetic gas treatment system includes a liquid replacement system (e.g., liquid replacement system 416 of FIG. 4). In some examples, the liquid replacement system may be included when the waste anesthetic gas treatment system includes a hybrid PCO/neutralization reactor. In alternate examples, the liquid replacement system may be fluidly coupled to a standalone neutralization reactor (e.g., neutralization reactor 334 of FIG. 3). Herein, method 1000 refers to a neutralization reactor may refer to a standalone neutralization reactor or a hybrid PCO/neutralization reactor. Method 1000 may occur in response to the controller determining that a pH of the neutralization solution is less than or equal to a threshold pH (e.g., YES at step 806 of method 800).

At 1002, method 1000 determines if a self-sealing cartridge includes fresh neutralization solution. Fresh neutralization solution includes basic species to react with acid byproducts to produce neutral salts. A pH of the fresh neutralization solution may be greater than 7.5. If method 1000 determines that the self-sealing cartridge does not include fresh neutralization solution (NO), method 1000 continues to 1012 and includes alerting a user to replace the self-sealing fluid cartridge. Method 1000 returns.

If method 1000 determines that the self-sealing cartridge does include fresh neutralization solution (YES), method 1000 proceeds to 1004 and includes opening a liquid shutoff valve. The liquid shut off valve may be positioned in a fluid passage between a bidirectional pump of the liquid replacement system and the neutralization reactor. Opening the liquid shutoff valve may enable fluid flow between the neutralization reactor and the liquid replacement system. At 1006, method 1000 includes activating the bidirectional pump in the first direction. Activating the bidirectional pump in the first direction may pump used (e.g., pH ≤ 7.5) neutralization solution from the neutralization reactor to a receiving compartment of the self-sealing fluid cartridge. The controller may activate the bidirectional pump in the first direction for a duration calibrated to empty the neutralization reactor of used neutralization solution.

At 1008, method 1000 includes activating the bidirectional pump in the second direction. Activating the bidirectional pump in the second direction may flow fresh neutralization solution from the self-sealing fluid cartridge to the neutralization reactor. The controller may activate the bidirectional pump in the second direction for a duration calibrated to full the neutralization reactor with a desired amount of fresh neutralization solution. At 1010, method 1000 includes closing the liquid shutoff valve. Closing the liquid shutoff valve may prevent further fluid flow between the neutralization reactor and the liquid replacement system. Method 1000 ends.

The technical effect of methods 700, 800 and 1000 is to degrade WAG gases to non-greenhouse gases and to neutralize acid gas byproducts. The methods may monitor and control the PCO and neutralization reactions to prevent introduction of harmful gases (e.g., acid gases or untreated WAG) to an enclosed space where users are present. Treating WAG gases by PCO may prevent introduction of greenhouse gases to the environment as a result of patient anesthesia. PCO may use readily available and abundant materials (e.g., TiO₂ and UV light) to degrade WAG gas to non-greenhouse gases.

The disclosure also provides support for a method for treating waste anesthetic gas, comprising: flowing waste anesthetic gas into an inlet of a photocatalytic oxidizing reactor, operating one or more ultraviolet lamps of the photocatalytic oxidizing reactor to trigger oxide catalyzed photodecomposition of the waste anesthetic gas and forming acidic byproducts, and flowing an output of the photocatalytic oxidizing reactor, including the acidic byproducts into a neutralization reactor. In a first example of the method, the photocatalytic oxidizing reactor is a packed bed photocatalytic oxidizing reactor including wide bandgap semiconductor oxide coated pellets packed around the one or more ultraviolet lamps. In a second example of the method, optionally including the first example, the method further comprises: flowing waste anesthetic gas through a flow sensor positioned upstream of the photocatalytic oxidizing reactor and in response to an output of the flow sensor indicating a flow rate above a threshold flow rate directing the waste anesthetic gas to an input of a gas accumulator positioned downstream of the flow sensor and upstream of the photocatalytic oxidizing reactor. In a third example of the method, optionally including one or both of the first and second examples, the neutralization reactor includes an aqueous solution configured to neutralize the acidic byproducts and a pH sensor configured to measure a pH of the aqueous solution. In a fourth example of the method, optionally including one or more or each of the first through third examples, the method further comprises: monitoring the pH of the aqueous solution and in response to the monitored pH less than or equal to 7.5, generate an alert. In a fifth example of the method, optionally including one or more or each of the first through fourth examples, the method further comprises: monitoring an outlet of the neutralization reactor for breakthrough waste anesthetic gas and/or acidic gases, and in response to presence of breakthrough waste anesthetic gas and/or acidic gases divert flow of waste anesthetic gas from the photocatalytic oxidizing reactor to a scavenging system and generate an alert. In a sixth example of the method, optionally including one or more or each of the first through fifth examples, the method further comprises: measuring a concentration of the acidic byproducts output from the photocatalytic oxidizing reactor. In a seventh example of the method, optionally including one or more or each of the first through sixth examples, the method further comprises: increasing an operating power of the one or more ultraviolet lamps in response to the measured concentration of the acidic byproducts being below a threshold concentration.

The disclosure also provides support for a waste anesthetic gas treatment system, comprising: a gas accumulator configured to receive waste anesthetic gas and output waste anesthetic gas at or above a threshold pressure, and a packed bed photocatalytic oxidizing reactor including oxide coated pellets packed around one or more ultraviolet lamps, and configured to receive the output of the gas accumulator. In a first example of the system, the gas accumulator includes a compressor and an accumulator tank fluidly coupled to the compressor, the accumulator tank positioned directly downstream of the packed bed photocatalytic oxidizing reactor. In a second example of the system, optionally including the first example, the packed bed photocatalytic oxidizing reactor further includes an ultraviolet sensor. In a third example of the system, optionally including one or both of the first and second examples, the system further comprises: a neutralization reactor configured to receive decomposition gas byproducts from an output of the packed bed photocatalytic oxidizing reactor. In a fourth example of the system, optionally including one or more or each of the first through third examples, the neutralization reactor includes an aqueous solution configured to neutralize acidic species of the decomposition gas byproducts. In a fifth example of the system, optionally including one or more or each of the first through fourth examples, the packed bed photocatalytic oxidizing reactor further includes an aqueous solution in which the oxide coated pellets are suspended, the aqueous solution configured to neutralize acidic byproducts produced by decomposition of the waste anesthetic gas.

The disclosure also provides support for a waste anesthetic gas treatment system, comprising: a photocatalytic oxidizing reactor including one or more ultraviolet lamps, and configured to receive waste anesthetic gas and output decomposition gas byproducts, a neutralization reactor fluidly coupled to an outlet of the photocatalytic oxidizing reactor, a valve coupling an inlet of the waste anesthetic gas treatment system to the photocatalytic oxidizing reactor, to an accumulator, and to an outlet of the waste anesthetic gas treatment system, a chemical sensor positioned at an outlet of the neutralization reactor, and a controller communicatively coupled to the valve and the chemical, and including instructions stored on non-volatile memory, the instructions executable to: monitor an output of the chemical sensor, in response to the output of the chemical sensor actuating the valve to direct flow of the waste anesthetic gas. In a first example of the system, the output of the chemical sensor indicates a concentration of breakthrough gas. In a second example of the system, optionally including the first example, the instructions further include to actuate the valve to direct waste anesthetic gas away from the accumulator and the photocatalytic oxidizing reactor and towards the outlet of the waste anesthetic gas treatment system in response to the output of the chemical sensor indicating the concentration of waste anesthetic gas above a breakthrough concentration. In a third example of the system, optionally including one or both of the first and second examples, the photocatalytic oxidizing reactor includes an ultraviolet transparent tube wound around at least one of the one or more ultraviolet lamps and ultraviolet transmitting fiber optics positioned in a lumen of the ultraviolet transparent tube, an inner surface of the ultraviolet transparent tube coated with an oxide catalyst. In a fourth example of the system, optionally including one or more or each of the first through third examples, the neutralization reactor includes a cooling system and a temperature sensor configured to monitor a temperature of coolant flowing through the cooling system. In a fifth example of the system, optionally including one or more or each of the first through fourth examples, the instructions further include to increase a power of the cooling system in response to an output of the temperature sensor indicating the temperature of the coolant is above 50 °C.

As used herein, an element or step recited in the singular and preceded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

FIGS. 5-6 show example configurations with relative positioning of the various components. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space there-between and no other components may be referred to as such, in at least one example. As yet another example, elements shown above/below one another, at opposite sides to one another, or to the left/right of one another may be referred to as such, relative to one another. Further, as shown in the figures, a topmost element or point of element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A method for treating waste anesthetic gas, comprising:
flowing (710) waste anesthetic gas into an inlet of a photocatalytic oxidizing reactor;
operating one or more ultraviolet lamps of the photocatalytic oxidizing reactor to trigger oxide catalyzed photodecomposition of the waste anesthetic gas and forming acidic byproducts; and
flowing (722) an output of the photocatalytic oxidizing reactor, including the acidic byproducts into a neutralization reactor.

2. The method of claim 1, wherein the photocatalytic oxidizing reactor is a packed bed photocatalytic oxidizing reactor including wide bandgap semiconductor oxide coated pellets packed around the one or more ultraviolet lamps.

3. The method of claim 1, further comprising flowing waste anesthetic gas through a flow sensor positioned upstream of the photocatalytic oxidizing reactor and in response to an output of the flow sensor indicating a flow rate above a threshold flow rate directing the waste anesthetic gas to an input of a gas accumulator positioned downstream of the flow sensor and upstream of the photocatalytic oxidizing reactor.

4. The method of claim 1, wherein the neutralization reactor includes an aqueous solution configured to neutralize the acidic byproducts and a pH sensor configured to measure a pH of the aqueous solution, and the method further comprising monitoring the pH of the aqueous solution and in response to the monitored pH less than or equal to 7.5, generate an alert.

5. The method of claim 1, further comprising monitoring an outlet of the neutralization reactor for breakthrough waste anesthetic gas and/or acidic gases, and in response to presence of breakthrough waste anesthetic gas and/or acidic gases divert flow of waste anesthetic gas from the photocatalytic oxidizing reactor to a scavenging system and generate an alert.

6. The method of claim 1, further comprising measuring a concentration of the acidic byproducts output from the photocatalytic oxidizing reactor, and further comprising increasing an operating power of the one or more ultraviolet lamps in response to the measured concentration of the acidic byproducts being below a threshold concentration.

7. A waste anesthetic gas treatment system, comprising:
a gas accumulator (313) configured to receive waste anesthetic gas and output waste anesthetic gas at or above a threshold pressure; and
a packed bed photocatalytic oxidizing reactor (316) including oxide coated pellets (328) packed around one or more ultraviolet lamps (330b), and configured to receive the output of the gas accumulator.

8. The waste anesthetic gas treatment system of claim 7, wherein the gas accumulator includes a compressor and an accumulator tank fluidly coupled to the compressor, the accumulator tank positioned directly downstream of the packed bed photocatalytic oxidizing reactor.

9. The waste anesthetic gas treatment system of claim 7, wherein the packed bed photocatalytic oxidizing reactor further includes an ultraviolet sensor.

10. The waste anesthetic gas treatment system of claim 7, wherein the packed bed photocatalytic oxidizing reactor further includes an aqueous solution in which the oxide coated pellets are suspended, the aqueous solution configured to neutralize acidic byproducts produced by decomposition of the waste anesthetic gas.

11. The waste anesthetic gas treatment system of claim 7, further comprising a valve coupling an inlet of the waste anesthetic gas treatment system to the packed bed photocatalytic oxidizing reactor, to the gas accumulator, and to an outlet of the waste anesthetic gas treatment system;
a chemical sensor positioned at an outlet of a neutralization reactor; and
a controller communicatively coupled to the valve and the chemical sensor, and including instructions stored on non-volatile memory, the instructions executable to:
monitor an output of the chemical sensor;
in response to the output of the chemical sensor actuating the valve to direct flow of the waste anesthetic gas.

12. The waste anesthetic gas treatment system of claim 11, wherein the output of the chemical sensor indicates a concentration of breakthrough gas and wherein the instructions further include to actuate the valve to direct waste anesthetic gas away from the gas accumulator and the packed bed photocatalytic oxidizing reactor and towards the outlet of the waste anesthetic gas treatment system in response to the output of the chemical sensor indicating the concentration of waste anesthetic gas above a breakthrough concentration.

13. The waste anesthetic gas treatment system of claim 7, wherein the packed bed photocatalytic oxidizing reactor includes an ultraviolet transparent tube wound around at least one of the one or more ultraviolet lamps and ultraviolet transmitting fiber optics positioned in a lumen of the ultraviolet transparent tube, an inner surface of the ultraviolet transparent tube coated with an oxide catalyst.

14. The waste anesthetic gas treatment system of claim 7, further comprising a neutralization reactor configured to receive decomposition gas byproducts from an output of the packed bed photocatalytic oxidizing reactor, and wherein the neutralization reactor includes an aqueous solution configured to neutralize acidic species of the decomposition gas byproducts, and wherein the neutralization reactor includes a cooling system and a temperature sensor configured to monitor a temperature of coolant flowing through the cooling system.

15. The waste anesthetic gas treatment system of claim 14, further comprising a controller communicatively coupled to the cooling system, and including instructions stored on non-volatile memory, the instructions executable to:
increase a power of the cooling system in response to an output of the temperature sensor indicating the temperature of the coolant is above 50 °C.
